# EUROPEAN PATENT APPLICATION

(11) **EP 3 795 090 A1**
(43) Date of publication of application: **24.03.2021**
(21) Application number: 19382814.2
(22) Date of filing: 23.09.2019
(51) Int. Cl.: A61B 17/00, A61F 2/06

(54) **PATCH DEPLOYMENT DEVICE**

(71) Applicant: Institut Químic de Sarrià CETS Fundació Privada, 08017 Barcelona (ES); Hospital Clinic De Barcelona, 08036 Barcelona (ES)
(72) Inventor: Aranda Gil, Alejandro, 08017 Barcelona (ES); García Granada, Andrés-Amador, 08017 Barcelona (ES); Martorell López, Jordi, 08017 Barcelona (ES); Riambau Alonso, Vicente, 08036 Barcelona (ES)
(74) Representative: Carpmaels & Ransford LLP

(57) **Abstract**

The present invention provides a patch deployment device, the device comprising a pusher wire having a proximal end and a distal end; a plurality of deployment wires, wherein each deployment wire: has a first end and a second end, and the first end and the second end are connected to the distal end of the pusher wire; and is configured to be in an unexpanded state when positioned and constrained within a catheter, and configured to self-expand into an expanded state when positioned beyond a distal end of the catheter and not constrained, wherein in the expanded state at least a portion of the wire is positioned substantially within a plane that is substantially perpendicular to the longitudinal axis of the pusher wire and has an asymmetric form when viewed along a direction parallel to the longitudinal axis of the pusher wire.

## Description

The present invention relates to a patch deployment device for deploying a patch within a patient, in particular for deploying a patch to damaged tissue within a patient. The present invention further relates to a patch deployment system comprising the patch deployment device and the patch to be deployed and/or a funnel to be used with the patch deployment device. The present invention further relates to a method of applying a patch.

Positioning patches within the body is useful for a range of medical procedures. Patches can be used to deliver a therapeutic agent or they can be used for repairing damaged tissue. For example, it has recently been proposed to treat tears in the aorta by applying a patch over the tear, thus stopping flow of blood out of the tear and a possible aortic dissection resulting from the tear. In order to apply a patch in a minimally invasive way, it needs to be introduced into the body through as small an incision as possible and then successfully and securely applied to the affected area. The present invention addresses this need.

The present invention provides a patch deployment device, the device comprising a pusher wire having a proximal end and a distal end; a plurality of deployment wires, wherein each deployment wire; (i) has a first end and a second end, and the first end and second end are connected to the distal end of the pusher wire; and (ii) is configured to be in an unexpanded state when positioned and constrained within a catheter, and configured to self-expand into an expanded state when positioned beyond a distal end of the catheter and not constrained, wherein in the expanded state at least a portion of the wire is positioned within a plane that is substantially perpendicular to the longitudinal axis of the pusher wire and has an asymmetric form when viewed along a direction parallel to the longitudinal axis of the pusher wire.

This patch deployment device is able to adopt an unexpanded configuration for ease of insertion into the patient and then it is able to adopt an expanded position when at the location where the patch is to be applied. This minimises the trauma associated with applying a patch to an internal surface of a patient.

Further, each deployment wire extending between a first end and a second end, where the ends are connected to the distal end of the pusher wire, as well as having an asymmetric form in-between these ends, results in the deployment wire experiencing asymmetric forces when self-expanding. This results in a rotational aspect to the deployment of the device and so reduces the amount of headspace required around the distal end of the catheter when expanding the patch deployment device. In other words, the presence of an asymmetric loop-like structure results in asymmetric forces on the deployment wire and thus corresponding asymmetric movement when the deployment wires self-expand. This allows the deployment wires to move from the unexpanded state to the expanded state in an efficient manner. This means that the free volume required around the distal end of the catheter for the expansion to occur is reduced which makes the device easier to use within the confined spaces within the body of the patient.

In general, the patch deployment device is a device that is capable of being used with a patch, such as a therapeutic patch, and can be used to deploy this patch to the required site within the body of a patient.

The pusher wire is a wire that is capable of transmitting forces from its proximal end to its distal end. Such forces can be a pushing force or pulling force applied to the proximal end of the pusher wire by the user of the patch deployment device. A typical user of the patch deployment device could be the surgeon conducting a procedure involving the application of the patch from the patch deployment device.

As used herein, the terms "proximal" and "distal" are used relative to the user of the patch deployment device, i.e. the surgeon. In this way, the proximal part of a component is closer to the user than the distal part of the component when in use.

As noted above, the pusher wire has a proximal end and a distal end. These two ends represent the far extremities of the wire. The wire can be made of any suitable material for transmitting forces from one of its ends to the other. A possible material for the pusher wire is stainless steel. Another possible material for the pusher wire is a nickel-titanium alloy, such as Nitinol. Further possible materials for the pusher wire include aluminium or any suitable metallic alloy.

The patch deployment device has a plurality of deployment wires. Deployment wires are wires that are involved in the deployment action performed by the device. In particular, the deployment wires can be directly in contact with the patch that is being deployed. The deployment wires each have the form of a wire. That is, each of the deployment wires has an elongate form.

The deployment wires can be made of any material that is capable of self-expanding in the patient. In other words, any material that can be constrained within a catheter to then elastically expand into a neutral expanded state. Such materials include elastic, superelastic and shape-memory materials, such as some plastics, metallic alloys or composite materials.

Each of the plurality of deployment wires has a first end and a second end. The first end and the second end are the respective limits along the length of the wire. The first end and the second end of each deployment wire is connected to the distal end of the pusher wire. When referring to the respective ends being connected to other components, this is considered to encompass a portion at the end of the respective components being connected to a referenced component. In other words, a portion adjacent the first end and a portion adjacent the second end of the deployment wire are connected to a portion adjacent the distal end of the pusher wire. A portion adjacent to the end relates to a portion that extends no more than 10% of the total length of the wire away from the respective ends, preferably no more than 5%, even more preferably no more than 2%.

Since the first and the second end of each deployment wire is connected to the distal end of the pusher wire, each deployment wire has a loop-like form where it is connected at the first end at the distal end of the pusher wire, it then extends along its length away from the pusher wire and then returning to be connected at the second end to the distal end of the pusher wire. Having two ends of each deployment wire connected to the distal end of the pusher wire increases the stability of the deployment wire extending between the two ends and thus increases the stability of the patch application procedure where the deployment wire is used to apply a force to the patch against an application site in the patient in order to assist adhering the patch to the required application site.

The connection between the first end and the second end of the deployment wires and the distal end of the pusher wire can be achieved by any suitable means. The connection may be a connection provided by an adhesive. The deployment wires may be tied or welded, or soldered to the distal end of the pusher wire. The connection may be provided by a compression fitting such as a sleeve around the ends of the deployment wires at the distal end of the pusher wire. In this way, the sleeve is holding the ends of the pusher wire against the distal end of the pusher wire and thus securing them in place.

Each of the deployment wires is configured to be in an unexpanded state when positioned and constrained within a catheter. The deployment wires are configured to self-expand into an expanded state when positioned beyond a distal end of the catheter and not constrained. In this manner, the plurality of deployment wires can adopt a configuration so that they can fit within a catheter when constrained to do so, but then return to a neutral configuration when they are not constrained. In this way, the neutral state (i.e. a state in which the plurality of deployment wires are not experiencing any external forces, beyond those used to connect the ends of the deployment wires to the distal end of the pusher wire) is the so-called expanded state. In the expanded state, the plurality of deployment wires are in the configuration utilised for applying the patch to an application site within the patient. The term "unexpanded state" is used to contrast to the expanded state but refers to a state where the plurality of deployment wires experience an external constraining force such as by being contained by the inner walls of a catheter.

The ability of the deployment wires to self-expand into the expanded state is based on the deployment wires ability to be elastically deformed so as to be in the unexpanded state when constrained. The deployment wires can then elastically return to an expanded state when that external force is removed. In this way, the plurality of deployment wires which are positioned in the catheter in a constrained configuration can move to the expanded state when the plurality of deployment wires are pushed out of the catheter, i.e. beyond a distal end of the catheter, such that they are no longer constrained by external forces.

A catheter is a tube which can be used with the patch deployment device, within which the plurality of deployment wires and the pusher wire can be placed. The catheter may have any suitable form and may be a commercial catheter. In particular, the catheter should be made of a suitable material that can be used within the body of a patient. The catheter has an opening at its distal end so that the plurality of deployment wires may be pushed out of the catheter.

A plurality of catheters may be used with the patch deployment device. For example, the patch deployment device can be used with two catheters. In particular, the patch deployment device can be used with an inner catheter, referred to herein as an applier catheter, and an outer catheter, referred to herein as a guider catheter. The patch deployment device can be constrained within the applier catheter to be in its unexpanded state and can be deployed out of the applier catheter to be in its expanded state. The guider catheter can assist in moving the applier catheter to the required application location. The guider catheter may have a steerable tip to further assist with reaching the required application location.

When a deployment wire is in the unexpanded state it is configured such that the maximum distance of the deployment wire from the longitudinal axis of the pusher wire is less than the maximum distance in the expanded state. In this way, the deployment wires can fit within the catheter.

When the deployment wires are in an expanded state, at least a portion of each of the deployment wires is positioned within a plane that is substantially perpendicular to the longitudinal axis of the pusher wire and each of the deployment wires has an asymmetric form when viewed along a direction parallel to longitudinal axis of the pusher wire. In this manner, the plurality of deployment wires form a surface substantially perpendicular to the longitudinal axis of the pusher wire which can then be used to press a patch onto a patch application site in the patient. Thus, this arrangement assists in placing the patch securely where it is required.

The portion of each deployment wire that is positioned within a plane that is substantially perpendicular to the longitudinal axis of the pusher wire may be at least 60% of the total length of the deployment wire, preferably at least 70%, more preferably at least 80%, even more preferably at least 90%.

The longitudinal axis of the pusher wire is the axis that runs along the length of the pusher wire. The deployment wires are perpendicular to the longitudinal axis relative to the direction of the longitudinal axis when it intersects the plane containing the portion of the deployment wire referred to above. When the plane is not intersected by the pusher wire itself, the reference to the longitudinal axis of the pusher wire includes a projection of the longitudinal axis of the pusher wire beyond the distal end based on the direction of the longitudinal axis at the distal end of the pusher wire.

At least a portion of each deployment wire is positioned substantially within a plane that is substantially perpendicular to the longitudinal axis of the pusher wire when in the expanded state. In this manner, the deployment wires in this portion are planar to the extent that they provide a sufficiently flat surface for applying the patch to an application site. Further, this plane is substantially perpendicular to the longitudinal axis, in other words, it is within 10° of perpendicular to the longitudinal axis, preferably within 5°, preferably within 2°.

The portion of the deployment wire that is positioned substantially within a plane that is substantially perpendicular to the longitudinal axis of the pusher wire also has an asymmetric form. In other words, all of the portion of the deployment wire that is positioned substantially within a plane that is substantially perpendicular to the longitudinal axis of the pusher wire has an asymmetric form. This asymmetric form can be appreciated when viewing the configuration of the expanded deployment wire along a direction parallel to the longitudinal axis of the pusher wire. That is, the section of the longitudinal axis against which the perpendicular nature of the plane containing the portion of the wire is judged. When viewed in this direction the asymmetric form does not have any planes of symmetry or any axis of rotational symmetry. The asymmetric nature results in the asymmetric forces that result in a rotational aspect to the expansion of the deployment wire from the unexpanded state to the expanded state.

As noted above, the plurality of deployment wires can be in the unexpanded state within the catheter and then be in an expanded state when positioned beyond the distal end of the catheter and no longer constrained. The plurality of deployment wires may be moved within the catheter by moving the pusher wire. The pusher wire may be movably positioned within the catheter in order to achieve movement of the pusher wire relative to the catheter.

The catheter may be a steerable catheter. In this way, the distal end of the catheter may be directed relative to the rest of the catheter so as to more easily position the distal end of the catheter in relation to the proposed application site onto which the patch is intended to be deployed.

The size of the catheter, i.e. its outer diameter, is chosen to be suitable for containing the deployment device as well as being able to be used in the required medical application. The catheter may have a size given in French gauge of from 2 to 97, preferably 4 to 32, more preferably 12 to 14. The catheter may have an inner diameter that is at least 1 less in terms of French gauge compared to its outer diameter. The catheter may have an inner diameter given in French gauge of from 1 to 96, preferably 3 to 31, more preferably 11 to 13.

One unit of French gauge is equivalent to 1/3 mm. Therefore, 3 French is equivalent to a diameter of 1 mm.

When two catheters are used, the inner catheter, also referred to as the applier catheter, may have the sizes mentioned above, while the outer catheter, also referred to as the guider catheter, may have an outer diameter size that is 2 or 4 more in terms of French gauge than the applier catheter's outer diameter. The inner diameter of the guider catheter may be at least 1 less in terms of French gauge than the outer diameter of the guider catheter. The guider catheter may have a size (outer diameter) given in French gauge of from 4 to 99, preferably 6 to 34, more preferably 14 to 18. The catheter may have an inner diameter that is at least 1 less in terms of French gauge compared to its outer diameter. The catheter may have an inner diameter given in French gauge of from 3 to 98, preferably 5 to 33, more preferably 13 to 17.

The catheters have a circular cross-sectional shape.

Each of the deployment wires of the plurality of deployment wires may have the same form in the expanded state. In other words, each of the deployment wires may be arranged into the same shape in the expanded state. Even though the deployment wires have the same shape they are not coincident and so will be positioned at different locations around the pusher wire. Each of the deployment wires having the same form contributes to the consistent behaviour when applying the patch to the required location.

The plurality of deployment wires may be arranged so as to be evenly distributed around the longitudinal axis of the pusher wire when in the expanded state. The even distribution of the pusher wires again contributes to the consistent performance in applying a patch to the required location regardless of the angular orientation of the deployment device. Evenly distributed means that each deployment wire of the plurality is positioned at regular angular intervals around the longitudinal axis of the pusher wire.

The asymmetric form of each deployment wire may comprise curved portions. In particular, the asymmetric form may comprise a first curved portion and a second curved portion wherein the first curved portion and the second curved portion are connected via a connecting portion. The connecting portion may also be curved.

The first curved portion may overlap the second curved portion in a circumferential direction around the longitudinal axis of the pusher wire. In other words, when a path is followed in a circumferential manner around the longitudinal axis of the pusher wire, where the path has a constant radial distance from the longitudinal axis of the pusher wire, it first passes through the first curved portion and then the second curved portion.

The first curved portion may be convex. A convex portion is one which protrudes away from the centre of the shape. The second curved portion may be concave. A concave portion is one that protrudes towards the centre of the shape. Reference to a shape here refers to the shape formed by the deployment wire. Having one concave portion and one convex portion joined by a connecting portion provides a crescent-like shape that requires a relatively small volume in order to move from the unexpanded state within a catheter to the expanded state outside of the catheter. The connecting portion may be the portion of deployment wire where the deployment wire transitions from being a concave shape to a convex shape.

The first curved portion may comprise a minimum radius of curvature that is less than the minimum radius of curvature of the second curved portion. In other words, at some point along the first curved portion there is a minimum radius of curvature and that value of radius of curvature is less than the point along the second curved portion that has a minimum radius of curvature for the second curved portion. Conversely, the first curved portion may comprise a minimum radius of curvature that is more than the minimum radius of curvature of the second curved portion. In other words, at some point along the first curved portion there is a minimum radius of curvature and that value of radius of curvature is more than the point along the second curved portion that has a minimum radius of curvature for the second curved portion. This increases the asymmetric nature of the deployment wire and this contributes to the asymmetric forces that are experienced when the deployment wire moves from the unexpanded to the expanded state, thus assisting an efficient deployment that requires less volume in order for the deployment to occur. In relation to the radius of curvature it could also be said that the first curved portion has a tighter curve than the second curved portion. Conversely, it could also be said that the second curved portion has a tighter curve than the first curved portion.

The plurality of deployment wires may be arranged such that there is overlap in a direction substantially parallel to the longitudinal axis of the pusher wire between different deployment wires. This can help increase the stability of the device in the expanded state when it is used to apply a patch to a required location. When there is a first curved portion and a second curved portion the first curved portion of one deployment wire may overlap the second curved portion of an adjacent deployment wire in a direction substantially parallel to the longitudinal axis of the pusher wire. Further, the first curved portion of one deployment wire may overlap the second curved portion of an adjacent deployment wire in a direction substantially parallel to the longitudinal axis of the pusher wire at a plurality of points, for example two points. Alternatively, or in addition, the first curved portion of one deployment wire may overlap the first curved portion of an adjacent deployment wire in a direction substantially parallel to the longitudinal axis of the pusher wire. Further, the first curved portion of one deployment wire may overlap the first curved portion of a plurality of adjacent deployment wires in a direction substantially parallel to the longitudinal axis of the pusher wire, for example, two adjacent deployment wires. Such overlap described herein can be true for all the deployment wires that are present so that each deployment wire is providing a further support to at least one other deployment wire.

The overlapping nature of the plurality of deployment wires may result in the plurality of deployment wires having an overall shape that is at least partially defined by each of the plurality of deployment wires. The plurality of deployment wires may, in combination, have the shape of a circle, i.e. the outer shape of the plurality of deployment wires is a circle when viewed along the longitudinal axis of the pusher wire. This provides consistent support to the patch around the perimeter of the plurality of deployment wires.

It is especially preferred that the plurality of deployment wires are formed from Nitinol. Nitinol is a nickel-titanium alloy that can exhibit shape memory and superelastic properties. The superelastic properties are particularly beneficial to the present invention since they allow the extensive deformation to elastically occur between the unexpanded state and the expanded state. This contributes to the ability of the deployment device to be contained within a small catheter. However, any material with sufficient elastic properties may be used with the present invention.

When Nitinol is used it is preferred that it exhibits its superelastic properties at body temperature. In this way, the deployment device will be exhibiting superelastic properties when used within the body. In order to ensure superelastic properties are exhibited the austenite finish temperature of the Nitinol should be below body temperature, i.e. below 37 °C. It is particularly preferred that the Nitinol has an austenite finish temperature of 28 °C or less. The austenite finish temperature may be -20 °C or greater, or -15 °C or greater. Typically, the austenite finish temperature is between 18 °C and 28 °C. In any case, the composition of the Nitinol should be chosen such that the Nitinol exhibits the required superelastic properties when used within the body.

The plurality of deployment wires may comprise any number of deployment wires that are suitable for the application. The plurality of deployment wires may comprise 20 deployment wires or fewer, 16 deployment wires or fewer, more preferably 12 deployment wires or fewer. A lower number of deployment wires makes it easier for the device to fit into smaller catheters.

The plurality of deployment wires may comprise two deployment wires or more, preferably four deployment wires or more, preferably 6 deployment wires or more. An increasing number of deployment wires improves the stability of the device when applying a patch to the required location.

A particularly favourable combination is to utilise eight deployment wires.

The plurality of deployment wires may each have a diameter of 0.127 mm (0.005 inches) or more. This assists the device in having a good level of stability when applying the patch to the required location.

The plurality of deployment wires may each have a diameter of 0.508 mm (0.02 inches) or less. This assists in the deployment device in having less internal forces when moving from the unexpanded state to the expanded state and being contained within a small catheter.

The diameter of each of the deployment wires relates to the maximum diameter of a cross-section of the wire that is perpendicular to the longitudinal axis of the wire. It is particularly preferred that the cross-section of the wire is circular.

The plurality of deployment wires may have a maximum diameter of 60 mm or less, preferably 35 mm or less, when each deployment wire of the plurality of deployment wires is in the expanded state. The plurality of deployment wires may have a maximum diameter of 5 mm or more when each deployment wire of the plurality of deployment wires is in the expanded state. A maximum diameter is the greatest diameter that spans across the centre of the plurality of deployment wires. The centre of the plurality of deployment wires is where the pusher wire is located. In general, the maximum diameter may be anything suitable for the required application, but 30 mm to 35 mm has been found to be particularly effective when used for applying patches to the required location.

The patch deployment device may be provided with the patch releasably attached to the plurality of deployment wires. Alternatively, the patch deployment device may be provided with the patch separate for attachment ahead of use. In any case, the releasable attachment of the patch to the plurality of deployment wires means that is can be released from the deployment wires when positioned and secured at the required location in the patient. The patch can be releasably attached to the plurality of deployment wires by using a bioadhesive. Such a bioadhesive may lose adhesion when positioned in the body and thus facilitate its removal from the deployment wires.

The patch may be attached to the plurality of deployment wires by a single thread, wherein the single thread is threaded through a plurality of loops associated with the patch, wherein both ends of the thread extend to the proximal end of the pusher wire. In this way, the user of the patch deployment device can hold both ends of the thread. By holding both ends of the thread, the patch is retained on the deployment wires. When the patch needs to be released, the user can let go of one end of the thread and pull the other end thus unthreading the wire through the loops and removing the means by which the patch was retained on the deployment wire assisting its release from the plurality of the deployment wires. The loops associated with the patch may be holes that are present in the patch. Instead of being weaved within the deployment wires, there can be a plurality of loops attached to the deployment wires through which the single thread is threaded. The thread may be a suture thread.

The patch deployment device may be inserted into the catheter with the assistance of a funnel. The funnel may be attached to the distal end of the catheter so that its narrowest end is closest to the distal end of the catheter, and it flares outwards in the distal direction. The deployment wires can then be drawn through the funnel such that they are gradually moved towards the unexpanded state from the expanded state by the decreasing diameter of the funnel. In this way, the funnel can contribute to the efficient packing of the deployment wires in the catheter. The funnel may be made from any suitable material including any suitable plastic.

Any suitable patch may be used as part of the patch deployment device, for example the patch may be bioabsorbable. The patch may be made from a polymeric material. The patch may be any suitable shape. However, an advantageous shape is circular shape so that it may be deployed at any angular orientation. Another advantageous shape is a rectangular shape, since patches are commonly deployed over linear tears, which can be efficiently covered by rectangular patches. The present invention is particularly suitable for use with the patch described in PCT application number PCT/EP2019/056358, which is incorporated herein by reference.

The present invention further provides a patch deployment system comprising the patch deployment device described herein and a separately provided patch and/or funnel and/or catheter.

The present invention also provides a method of applying a patch comprising the following steps: (i) providing the patch deployment device as described herein with a plurality of deployment wires in the unexpanded state within a catheter; (ii) inserting the catheter into a patient so as to position the distal end of the catheter at a patch application site; (iii) moving the pusher wire to move the plurality of deployment wires from the unexpanded state within the catheter to the expanded state beyond the distal end of the catheter; (iii) applying the patch to the patch application site; (iv) releasing the patch from the plurality of deployment wires; (v) moving the pusher wire to move the plurality of deployment wires from the expanded state to the unexpanded state; and (vi) removing the catheter from the patient.

This approach utilising the patch deployment device of the present invention advantageously requires a relatively small amount of headspace around the distal end of the catheter for deploying the patch deployment device within the patient and also suitably supports the patch when applying it to the patch application site. The patch application site can be any site in the patient that requires the application of a patch. The present invention is particularly suited for applying a patch to a tear within the aorta of the patient with a patch that is suitable for repairing that tear.

The present invention will now be described with reference to the following figures.
Figure 1 depicts a patch deployment system with the patch deployment device of the present invention;
Figure 2 depicts detail of the patch deployment device of Figure 1;
Figure 3 depicts the plurality of deployment wires of the patch deployment device of the present invention viewed along the longitudinal axis of the pusher wire;
Figure 4 depicts the plurality of deployment wires of Figure 3 viewed perpendicular to the longitudinal axis of the pusher wire;
Figure 5 depicts the patch deployment device of the present invention viewed along the longitudinal axis of the pusher wire;
Figure 6 depicts the patch deployment device of the present invention with a patch releasably attached;
Figure 7 depicts the patch deployment device of the present invention with a patch releasably attached and a funnel at the distal end of the catheter;
Figure 8 depicts a steerable catheter that is part of the patch deployment system of the present invention;
Figure 9 depicts the patch deployment device of Figure 8 where the deployment wires are being deployed;
Figure 10 depicts the patch deployment device of Figure 8, where the deployment wires are fully deployed;
Figure 11 schematically depicts a cross section of the aorta with a tear in its wall;
Figure 12 schematically depicts the patch deployment device of the present invention positioned adjacent a tear in the aorta wall;
Figure 13 schematically depicts the patch deployment device in the expanded configuration and a patch applied to the tear in the aorta wall;
Figure 14A and 14B depicts an alternative patch deployment system of the present invention with two catheters;
Figure 15 depicts an alternative embodiment of the plurality of deployment wires of the patch deployment device of the present invention viewed along the longitudinal axis of the pusher wire; and
Figure 16 depicts the plurality of deployment wires of Figure 15 viewed perpendicular to the longitudinal axis of the pusher wire.

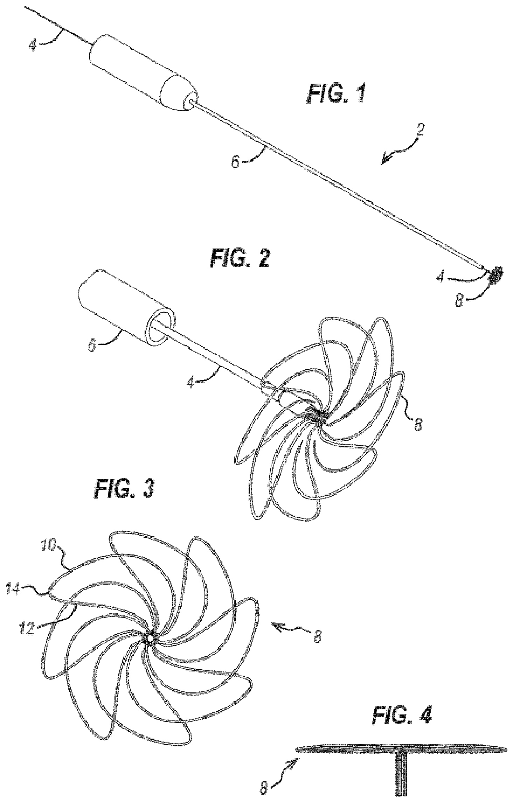

Figure 1 depicts a patch deployment device 2 of the present invention. The patch deployment device 2 has a pusher wire 4 that extends the fill length of catheter 6. There are a plurality of deployment wires 8 that are shown in the expanded state. The pusher wire 4 is connected to the deployment wires 8 at the distal end of the pusher wire 4. Further, the pusher wire 4 is shown protruding beyond the distal end of the catheter 6. This is shown in further detail in Figure 2.

The expanded configuration of the deployment wires 8 is shown in greater detail in Figures 3 and 4. The plurality of deployment wires is made up of 8 separate deployment wires. Each of these deployment wires has a first and second end which is connected to the distal end of the pusher wire 4. In this way, each deployment wire forms a loop-like structure when viewed along the longitudinal axis of the pusher wire. Each deployment wire has a portion that lies substantially in a plane that is substantially perpendicular to the longitudinal axis of the pusher wire 4. This portion has an asymmetric form similar to a crescent shape. The asymmetric form has a first curved portion 10 and a second curved portion 12 connected by a connecting portion 14. The first curved portion 10 overlaps the second curved portion 12 in a circumferential direction around the longitudinal axis of the pusher wire 4. Also, the first curved portion 10 has a tighter curve shape than the second curved portion 12. This emphasises the asymmetric form of the deployment wire in the plane that is perpendicular to the longitudinal axis of the pusher wire 4. The first curved portion 10 has a convex shape and the second curved portion 12 has a concave shape. Each of the first curved portions 10 overlap the second curved portion 12 of an adjacent deployment wire in a direction parallel to the longitudinal axis of the pusher wire 4. Overall, the plurality of deployment wires form a flower-like configuration where each deployment wire has the same form and is evenly distributed around the longitudinal axis of the pusher wire 4.

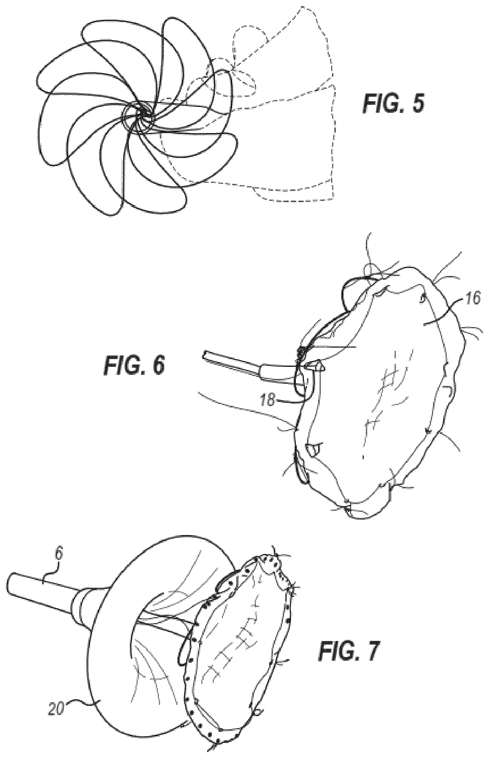

Figures 5 and 6 show the patch deployment device without a patch 16 attached and with a patch attached, respectively. The patch 16 is depicted as circular with a plurality of holes 18 in the patch 16 through which a single thread can be threaded in order to keep the patch 16 attached to the deployment device. The thread can be unthreaded from the holes from the proximal end of the device so as to release the patch 16 when it is adhered to the required application site.

Figure 7 depicts the patch deployment device with a patch attached and a funnel 20 positioned so as to assist the drawing of the plurality of deployment wires 8 into the catheter 6. The funnel 20 is positioned at the distal end of the catheter 6 with the flared portion further from the distal end than the narrow portion. The use of the funnel 20 assists moving the patch deployment wires 8 from the expanded state to the unexpanded state.

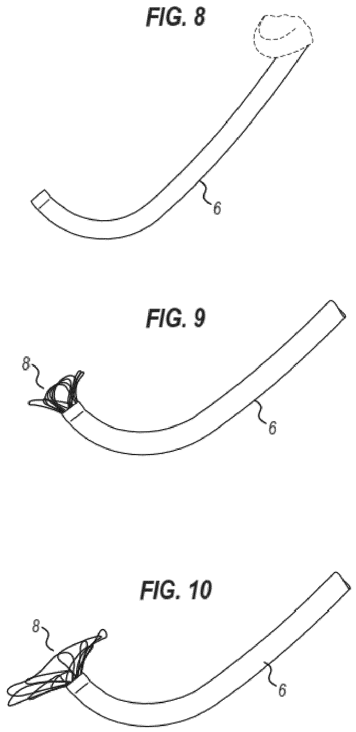

Figures 8, 9 and 10 depict the sequence of the plurality of deployment wires 8 being pushed out of catheter 6. In particular, it can be seen that the plurality of deployment wires 8 moves from the unexpanded state within the catheter 6 to an expanded state outside the catheter 6 without requiring a significant amount of headspace around the distal end of the catheter 6. This is a particularly efficient way of deploying a patch in a patient.

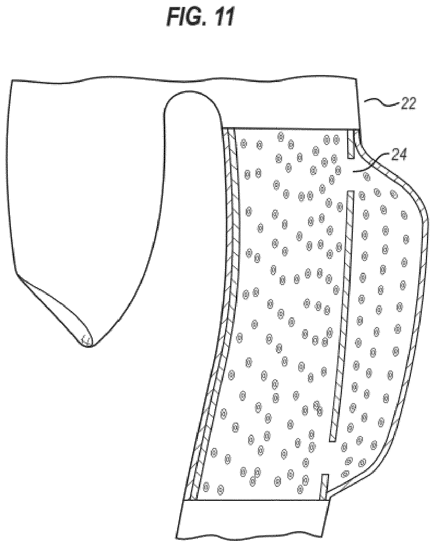

A procedure utilising the patch deployment device will now be described in relation to Figures 11, 12 and 13.

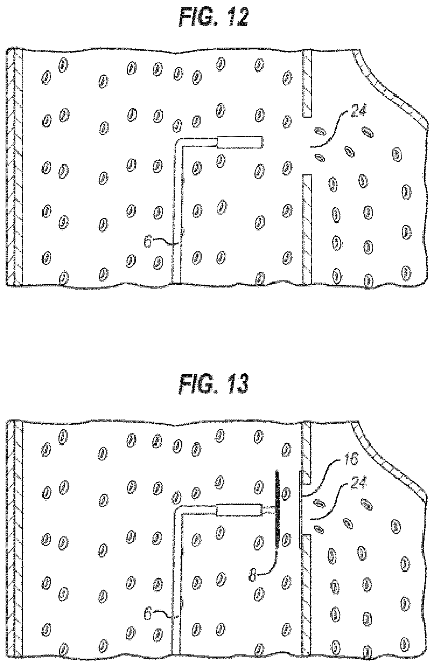

Figure 11 depicts part of the aorta 22 with a tear 24 in its wall. This results in blood flowing into a region known as a false lumen. Figure 12 shows a catheter 6 being positioned near the tear in the wall 24, i.e. at the patch application site. Figure 13 depicts the patch 16 having been applied to the tear 24 by being pressed onto the tear by the plurality of deployment wires 8. The patch deployment device can then be put into the unexpanded state and the catheter 6 removed from the aorta.

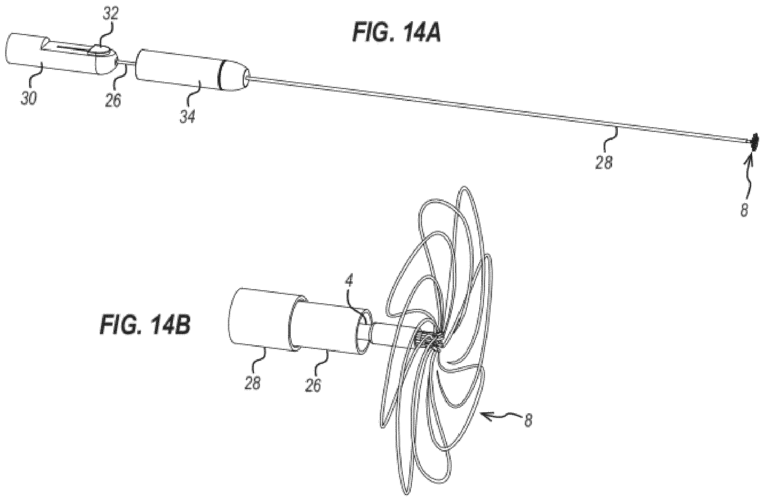

Figures 14A and 14B depict a further embodiment of the invention utilising an inner, applier catheter 26 with an associated handle 30 and an outer, guider catheter 28 with an associated handle 34. The pusher wire 4 is movably positioned within the applier catheter 26 and the applier catheter 26 is movably positioned within the guider catheter 28. The handle 30 associated with the applier catheter 26 has a slider 32 that is connected to the pusher wire 4 such that a distal movement of the slider 32, towards the far end of the applier catheter 26, results in a distal movement of the deployment wires 8 out of the distal end of the applier catheter and into the expanded state.

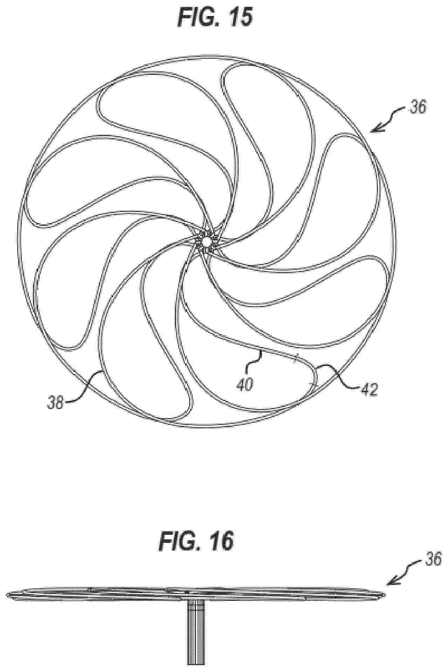

Figures 15 and 16 depict an alternative configuration of the deployment wires 36 in the expanded state. This configuration has a plurality of deployment wires where each deployment wire has the same form and is evenly distributed around the longitudinal axis of the pusher wire 4. Each of these deployment wires has a first and second end which is connected to the distal end of the pusher wire 4. In this way, each deployment wire forms a loop-like structure when viewed along the longitudinal axis of the pusher wire 4. Each deployment wire has a portion that lies substantially in a plane that is substantially perpendicular to the longitudinal axis of the pusher wire 4. This portion has an asymmetric form similar to a crescent shape. The asymmetric form has a first curved portion 38 and a second curved portion 40 connected by a connecting portion 42. The first curved portion 38 overlaps the second curved portion 40 in a circumferential direction around the longitudinal axis of the pusher wire 4. Also, the second curved portion 40 has a tighter curve shape than the first curved portion 38. This emphasises the asymmetric form of the deployment wire in the plane that is perpendicular to the longitudinal axis of the pusher wire 4. The first curved portion 38 has a convex shape and the second curved portion 40 has a concave shape. Each of the first curved portions 38 overlaps the second curved portion 40 of an adjacent deployment wire in a direction parallel to the longitudinal axis of the pusher wire 4. Further, each of the first curved portions 38 overlaps the first curved portions 38 of two adjacent deployment wires in a direction parallel to the longitudinal axis of the pusher wire 4, One of the adjacent deployment wires being the nearest deployment wire in the clockwise direction and the other adjacent deployment wire being the nearest deployment wire in the anticlockwise direction around the longitudinal axis of the pusher wire 4. Overall, the plurality of deployment wires form a flower-like configuration where the outer shape of the plurality of deployment wires is a circle.

The present invention has described with reference to specific embodiments. However, the invention is not limited by these specific embodiments and encompasses within the scope of the following claims.

The following list of embodiments forms part of the description.
1. A patch deployment device, the device comprising
   a pusher wire having a proximal end and a distal end;
   a plurality of deployment wires, wherein each deployment wire:
      (i) has a first end and a second end, and the first end and the second end are connected to the distal end of the pusher wire; and
      (ii) is configured to be in an unexpanded state when positioned and constrained within a catheter, and configured to self-expand into an expanded state when positioned beyond a distal end of the catheter and not constrained, wherein in the expanded state at least a portion of the wire is positioned substantially within a plane that is substantially perpendicular to the longitudinal axis of the pusher wire and has an asymmetric form when viewed along a direction parallel to the longitudinal axis of the pusher wire.
2. The patch deployment device of embodiment 1, wherein the plurality of deployment wires is movable from the unexpanded state within a catheter to the expanded state beyond the distal end of the catheter by moving the pusher wire.
3. The patch deployment device of any preceding embodiment, wherein each deployment wire of the plurality of deployment wires has the same form in the expanded state.
4. The patch deployment device of any preceding embodiment, wherein the plurality of deployment wires are arranged so as to be evenly distributed around the longitudinal axis of the pusher wire when in the expanded state.
5. The patch deployment device of any preceding embodiment, wherein the portion of the wire having an asymmetric form comprises a first curved portion and a second curved portion, wherein the first curved portion overlaps the second curved portion in a circumferential direction around the longitudinal axis of the pusher wire, and the first curved portion is convex and the second curved portion is concave, the first curved portion and the second curved portion being connected via a connecting portion.
6. The patch deployment device of embodiment 5, wherein the first curved portion comprises a minimum radius of curvature that is less than the minimum radius of curvature of the second curved portion.
7. The patch deployment device of embodiment 5, wherein the first curved portion comprises a minimum radius of curvature that is more than the minimum radius of curvature of the second curved portion.
8. The patch deployment device of any one of embodiments 5 to 7, wherein each deployment wire of the plurality of deployment wires is arranged such that the first curved portion of the deployment wire overlaps the second curved portion of an adjacent deployment wire in a direction substantially parallel to the longitudinal axis of the pusher wire.
9. The patch deployment device of any preceding embodiment, wherein the plurality of deployment wires comprises Nitinol.
10. The patch deployment device of embodiment 9, wherein the Nitinol has an austenite finish temperature of 28 °C or less.
11. The patch deployment device of any preceding embodiment, wherein the plurality of deployment wires comprises 20 deployment wires or fewer.
12. The patch deployment device of any preceding embodiment, wherein the plurality of deployment wires comprises 2 deployment wires or more.
13. The patch deployment device of any preceding embodiment, wherein the plurality of deployment wires comprises 8 deployment wires.
14. The patch deployment device of any preceding embodiment, wherein the plurality of deployment wires have a diameter of 0.127 mm or more.
15. The patch deployment device of any preceding embodiment, wherein the plurality of deployment wires have a diameter of 0.508 mm or less.
16. The patch deployment device of any preceding embodiment, wherein the plurality of deployment wires has a maximum diameter of 35 mm or less, when each deployment wire of the plurality of deployment wires is in the expanded state.
17. The patch deployment device of any preceding embodiment, further comprising a patch releasably attached to the plurality of deployment wires.
18. The patch deployment device of embodiment 17, wherein the patch is attached to the plurality of deployment wires by a single thread, wherein the single thread is threaded through a plurality of loops associated with the patch.
19. The patch deployment device of any preceding embodiment, wherein the catheter has a size of 18 Fr or less.
20. The patch deployment device of any preceding embodiment, wherein the catheter has a size of 12 Fr or greater.
21. A patch deployment system, wherein the patch deployment system comprises the patch deployment device of any one of embodiments 1 to 16, or embodiments 19 or 20 when dependent on any one of embodiments 1 to 16; and
   a patch, wherein the patch is attachable to the patch deployment device.
22. The patch deployment system of embodiment 21, further comprising the catheter, wherein the pusher wire can be movably positioned within the catheter
23. A patch deployment system, the patch deployment system comprising
   the patch deployment device of embodiments 17 or 18, or embodiments 19 or 20 when dependent on embodiment 17 or 18; and
   the catheter, wherein the pusher wire can be movably positioned within the catheter.
24. A patch deployment system, the patch deployment system comprising
   the patch deployment device of any one of embodiments 1 to 16, or embodiments 19 or 20 when dependent on any one of embodiments 1 to 16; and
   the catheter, wherein the pusher wire can be movably positioned within the catheter.
25. The patch deployment system of embodiment 24, further comprising a patch, wherein the patch is attachable to the patch deployment device.
26. The patch deployment system of any one of embodiments 21 to 25, further comprising a funnel for use when drawing the plurality of deployment wires into the catheter from the expanded state into the unexpanded state.
27. The patch deployment device of embodiment 17 or 18, or embodiments 19 or 20 when dependent on embodiments 17 or 18, or the patch deployment system of any one of embodiments 21, 22, 23, or 25, or embodiment 26 when dependent on any one of embodiments 21, 22, 23, or 25, wherein the patch is bioabsorbable.
28. The patch deployment of embodiment 17, 18 or 27, or embodiments 19 or 20 when dependent on embodiments 17 or 18, or the patch deployment system of any one of embodiments 21, 22, 23, 25, or 27, or embodiment 26 when dependent on any one of embodiments 21, 22, 23, or 25, wherein the patch further comprises a bioabsorbable adhesive.
29. A method of applying a patch comprising the following steps:
   (i) providing the patch deployment device of embodiment 17 with the plurality of deployment wires in the unexpanded state within the catheter;
   (ii) inserting the catheter into a patient so as to position the distal end of the catheter at a patch application site;
   (iii) moving the pusher wire to move the plurality of deployment wires from the unexpanded state within the catheter to the expanded state beyond the distal end of the catheter;
   (iv) applying the patch to the patch application site;
   (v) releasing the patch from the plurality of deployment wires;
   (vi) moving the pusher wire to move the plurality of deployment wires from the expanded state to the unexpanded state; and
   (vii) removing the catheter from the patient.

## Claims

1. A patch deployment device, the device comprising
a pusher wire having a proximal end and a distal end;
a plurality of deployment wires, wherein each deployment wire:
(iii) has a first end and a second end, and the first end and the second end are connected to the distal end of the pusher wire; and
(iv) is configured to be in an unexpanded state when positioned and constrained within a catheter, and configured to self-expand into an expanded state when positioned beyond a distal end of the catheter and not constrained, wherein in the expanded state at least a portion of the wire is positioned substantially within a plane that is substantially perpendicular to the longitudinal axis of the pusher wire and has an asymmetric form when viewed along a direction parallel to the longitudinal axis of the pusher wire.

2. The patch deployment device of claim 1, wherein the plurality of deployment wires is movable from the unexpanded state within a catheter to the expanded state beyond the distal end of the catheter by moving the pusher wire.

3. The patch deployment device of any preceding claim, wherein each deployment wire of the plurality of deployment wires has the same form in the expanded state.

4. The patch deployment device of any preceding claim, wherein the plurality of deployment wires are arranged so as to be evenly distributed around the longitudinal axis of the pusher wire when in the expanded state.

5. The patch deployment device of any preceding claim, wherein the portion of the wire having an asymmetric form comprises a first curved portion and a second curved portion, wherein the first curved portion overlaps the second curved portion in a circumferential direction around the longitudinal axis of the pusher wire, and the first curved portion is convex and the second curved portion is concave, the first curved portion and the second curved portion being connected via a connecting portion.

6. The patch deployment device of claim 5, wherein the first curved portion comprises a minimum radius of curvature that is less than the minimum radius of curvature of the second curved portion, or wherein the first curved portion comprises a minimum radius of curvature that is more than the minimum radius of curvature of the second curved portion.

7. The patch deployment device of claim 5 or claim 6, wherein each deployment wire of the plurality of deployment wires is arranged such that the first curved portion of the deployment wire overlaps the second curved portion of an adjacent deployment wire in a direction substantially parallel to the longitudinal axis of the pusher wire.

8. The patch deployment device of any preceding claim, wherein the plurality of deployment wires comprises Nitinol.

9. The patch deployment device of any preceding claim, wherein the plurality of deployment wires comprises 8 deployment wires.

10. The patch deployment device of any preceding claim, wherein the plurality of deployment wires have a diameter of from 0.127 mm to 0.508 mm.

11. The patch deployment device of any preceding claim, wherein the plurality of deployment wires has a maximum diameter of 35 mm or less when each deployment wire of the plurality of deployment wires is in the expanded state.

12. The patch deployment device of any preceding claim, further comprising a patch releasably attached to the plurality of deployment wires.

13. The patch deployment device of any preceding claim, wherein the catheter has a size of 18 Fr or less.

14. A patch deployment system, the patch deployment system comprising
the patch deployment device of any preceding claim; and
the catheter, wherein the pusher wire can be movably positioned within the catheter.

15. The patch deployment system of claim 14, further comprising a funnel for use when drawing the plurality of deployment wires into the catheter from the expanded state into the unexpanded state.
